# EUROPEAN PATENT SPECIFICATION

(11) **EP 4 058 013 B1**
(45) Date of publication and mention of the grant of the patent: **25.03.2026**
(21) Application number: 20821125.0
(22) Date of filing: 16.11.2020
(51) Int. Cl.: A61K 31/375, A61K 31/722, A61P 3/06, A61P 9/00, A61P 9/10

(54) **CHITOSAN FOR USE IN A METHOD OF PREVENTING OR TREATING A CARDIOVASCULAR DISEASE**
CHITOSAN ZUR VERWENDUNG IN EINEM VERFAHREN ZUR VORBEUGUNG ODER BEHANDLUNG EINER KARDIOVASKULÄREN ERKRANKUNG
CHITOSANE DESTINÉ À ÊTRE UTILISÉ DANS UN PROCÉDÉ DE PRÉVENTION OU DE TRAITEMENT D'UNE MALADIE CARDIOVASCULAIRE

(30) Priority: 15.11.2019 IT 201900021291
(43) Date of publication of application: 21.09.2022
(73) Proprietor: Certmedica International GmbH, 63741 Aschaffenburg (DE)
(72) Inventor: CORNELLI, Umberto, 20129 MILANO (IT)
(74) Representative: Cattaneo, Elisabetta
(86) International application number: PCT/EP2020/082264
(87) International publication number: WO 2021/094610

(56) References cited:
- US-A1- 2006 058 261
- US-A1- 2007 031 468
- BELCARO GIANNI ET AL: "Intestinal fat absorption shifting by polyglucosamine biopolymer: control of lipids and reduction of progression of early subclinical atherosclerosis", MINERVA GASTROENTEROLOGY, vol. 70, no. 1, 1 April 2024 (2024-04-01), XP093316037, ISSN: 2724-5985, DOI: 10.23736/S2724-5985.23.03539-8
- CORNELLI U ET AL: "Meta-analysis of studies on body weight and cholesterol reduction using the chitosan derivative polyglucosamine L112", GENERAL MEDICINE OPEN, vol. 2, no. 5, 1 January 2018 (2018-01-01), XP055715303, DOI: 10.15761/GMO.1000143

## Description

### FIELD OF THE INVENTION

The present invention relates to a chitosan having number average molecular weight in the range from 80 kDa to 350 kDa and a medical product comprising said chitosan, for use in a method of preventing or treating a cardiovascular disease, wherein chitosan is a linear polysaccharide composed of randomly distributed β-(1-4)-linked D-glucosamine (deacetylated unit) and N-acetyl-D-glucosamine (acetylated unit).

### BACKGROUND

Cardiovascular disease is a general term for conditions affecting the heart or blood vessels which include, among others, coronary artery diseases such as angina and myocardial infarction (commonly known as a heart attack), stroke, heart failure, hypertensive heart disease, rheumatic heart disease, and cardiomyopathy.

Cardiovascular diseases are usually associated with a build-up of fatty deposits inside the arteries (atherosclerosis) and an increased risk of blood clots.

US2007/0031468 discloses a method for embolizing a vascular site, comprising the introduction of an aqueous solution of acrylated chitosan for the deliberate embolization of a vascular duct.

Even though the exact cause of cardiovascular diseases are not fully known up to today, biological factors and habits that can increase the risk thereof, called "risk factors", are known, among which hypertension, smoking, diabetes, cholesterol, body weight, and family history (https://www.nhs.uk/conditions/cardiovascular-disease/).

Conversely, even if products and habits that may contribute to reduce the chances of getting them are known, the Applicant has noted that it is still felt the need of products capable of preventing and/or reducing the consequences of cardiovascular diseases.

### SUMMARY OF THE INVENTION

An object of the present invention is therefore the provision of a new product for preventing or treating a cardiovascular disease.

The invention is defined by the claims. Any subject-matter falling outside the scope of the claims is provided for information purposes only. Any references in the description to methods of treatment refer to the pharmaceutical composition and medicament according to the present invention for use in a method for treatment of the human or animal body by therapy. Said product is thus a chitosan, being a linear polysaccharide composed of randomly distributed β-(1→4)-linked D-glucosamine (deacetylated units) and N-acetyl-D-glucosamine (acetylated units), having a number average molecular weight in the range of 80 kDa to 350 kDa. Any reference to a reduction in arterial intima-media thickness and/or plaque density in blood vessels is to be understood as therapeutic.

Therefore, the present invention relates, in a first aspect, to a chitosan having number average molecular weight in the range from 80 kDa to 350 kDa, for use in a method of preventing or treating a cardiovascular disease, wherein chitosan is a linear polysaccharide composed of randomly distributed β-(1→4)-linked D-glucosamine (deacetylated unit) and N-acetyl-D-glucosamine (acetylated unit).

In a specific aspect, the present invention relates to a chitosan having number average molecular weight in the range from 80 kDa to 350 kDa, for in therapeutically reducing the arterial intima media thickness, wherein chitosan is a linear polysaccharide composed of randomly distributed β-(1→4)-linked D-glucosamine (deacetylated unit) and N-acetyl-D-glucosamine (acetylated unit).

In a further specific aspect, the present invention relates also to a chitosan having number average molecular weight in the range from 80 kDa to 350 kDa, for use in therapeutically controlling the plaque density in blood vessels, wherein chitosan is a linear polysaccharide composed of randomly distributed β-(1→4)-linked D-glucosamine (deacetylated unit) and N-acetyl-D-glucosamine (acetylated unit).

The present invention relates therefore to the use of a chitosan having number average molecular weight in the range from 80 kDa to 350 kDa for reducing the arterial intima media thickness and/or for controlling the plaque density in blood vessels, thus providing a product and method for preventing or treating a cardiovascular disease. That is, the present disclosure relates to a method for reducing the arterial intima media thickness and/or to controlling the plaque density in blood vessels comprising the use of a chitosan having number average molecular weight in the range from 80 kDa to 350 kDa.

The Applicant has indeed unexpectedly found out that a specific kind of chitosan, showing a number average molecular weight in the range from 80 kDa to 350 kDa, is capable of reducing the arterial intima media thickness and is also capable of controlling the plaque density in blood vessels, thus proving to be effective in preventing or treating a cardiovascular disease.

In a further aspect, the present invention relates also to a medical product comprising a chitosan having number average molecular weight in the range from 80 kDa to 350 kDa and at least one organic acid selected from the group consisting of ascorbic acid and tartaric acid, for use in a method of preventing or treating a cardiovascular disease.

The Applicant has noted that in a meta-analysis of clinical studies conducted with a particular chitosan in presence of organic acids (ascorbic and tartaric acids), a consistent body weight reduction was documented together with cholesterol reduction (Cornelli U, Belcaro G. Meta-analysis of studies on body weight and cholesterol reduction using the chitosan derivative poluglucosamine L112. Gen Med Open 2018; 2(5):1-4).

The Applicant has found out that by combining the chitosan according to the present invention with at least one organic acid selected from the group consisting of ascorbic acid and tartaric acid a medical product not only capable of reducing cholesterol and body weight, but also capable of reducing the arterial intima media thickness and of controlling the plaque density in blood vessels is obtained.

The Applicant therefore notes that such a medical product provides a further and improved product suitable for preventing or treating a cardiovascular disease, that contributes to reduce the arterial intima media thickness, the plaque density in blood vessels.

The present invention relates therefore to the use of a medical product comprising a chitosan having number average molecular weight in the range from 80 kDa to 350 kDa and at least one organic acid selected from the group consisting of ascorbic acid and tartaric acid, for reducing the arterial intima media thickness and/or for controlling the plaque density in blood vessels, thus providing a product and method for preventing or treating a cardiovascular disease, wherein chitosan is a linear polysaccharide composed of randomly distributed β-(1→4)-linked D-glucosamine (deacetylated unit) and N-acetyl-D-glucosamine (acetylated unit. That is, the present disclosure relates to a method for reducing the arterial intima media thickness and/or to controlling the plaque density in blood vessels comprising the use of a medical product comprising a chitosan having number average molecular weight in the range from 80 kDa to 350 kDa and at least one organic acid selected from the group consisting of ascorbic acid and tartaric acid, wherein chitosan is a linear polysaccharide composed of randomly distributed β-(1→4)-linked D-glucosamine (deacetylated unit) and N-acetyl-D-glucosamine (acetylated unit.

### BRIEF DESCRIPTION OF THE DRAWINGS

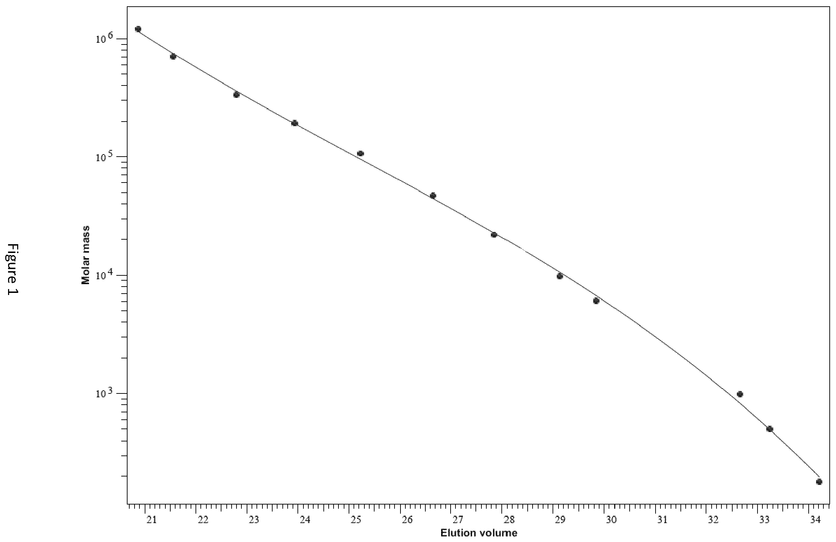

Figure 1 shows the pullulan calibration curve used for determining the number average molecular weight of the chitosan according to Example 1 by gel permeation chromatography.

### DETAILED DESCRIPTION OF THE INVENTION

The present invention relates, in a first aspect, to a chitosan having number average molecular weight in the range from 80 kDa to 350 kDa, for use in a method of preventing or treating a cardiovascular disease, wherein chitosan is a linear polysaccharide composed of randomly distributed β-(1→4)-linked D-glucosamine (deacetylated unit) and N-acetyl-D-glucosamine (acetylated unit.

In a specific aspect, the present invention relates to a chitosan having number average molecular weight in the range from 80 kDa to 350 kDa, for use in therapeutically reducing the arterial intima media thickness, wherein chitosan is a linear polysaccharide composed of randomly distributed β-(1→4)-linked D-glucosamine (deacetylated unit) and N-acetyl-D-glucosamine (acetylated unit. Preferably, said arterial intima media thickness is carotid and/or aorta intima media thickness.

In a further specific aspect, the present invention relates also to a chitosan having number average molecular weight in the range from 80 kDa to 350 kDa, for use in therapeutically reducing controlling the plaque density in blood vessels, wherein chitosan is a linear polysaccharide composed of randomly distributed β-(1→4)-linked D-glucosamine (deacetylated unit) and N-acetyl-D-glucosamine (acetylated unit.

The present invention relates therefore to the use of a chitosan having number average molecular weight in the range from 80 kDa to 350 kDa for reducing the arterial intima media thickness and/or for controlling the plaque density in blood vessels, thus providing a product and method for preventing or treating a cardiovascular disease. That is, the present disclosure relates to a method for reducing the arterial intima media thickness and/or to controlling the plaque density in blood vessels comprising the use of a chitosan having number average molecular weight in the range from 80 kDa to 350 kDa.

The Applicant has indeed unexpectedly found out that a specific kind of chitosan, showing a number average molecular weight in the range from 80 kDa to 350 kDa, is capable of reducing the arterial intima media thickness, notably in carotid and aorta, and is also capable of controlling the plaque density in blood vessels, thus proving to be effective in preventing or treating a cardiovascular disease.

In the context of the present application, the expression "chitosan" indicates a linear polysaccharide composed of randomly distributed β-(1→4)-linked D-glucosamine (deacetylated unit) and N-acetyl-D-glucosamine (acetylated unit). Chitosan is, in general, produced by deacetylation of chitin, which is the structural element in the exoskeleton of crustaceans (such as crabs and shrimp) and cell walls of fungi.

In the context of the present application, the expression "number average molecular weight" indicates the ordinary arithmetic mean or average of the molecular masses of the individual macromolecules of a polymer. In the present invention the number average molecular mass of chitosan can be determined by gel permeation chromatography.

The chitosan according to the present invention has a number average molecular weight in the range from 80 kDa to 350 kDa.

The determination of the number average molecular weight of chitosan may be carried out by gel permeation chromatography using calibration with pullulan standards, and with a solution 0.1 M NaCl / 0.1% by volume TFA aq. as eluent.

Preferably, the chitosan according to the present invention has a degree of deacethylation greater than or equal to 60% by moles, more preferably greater than or equal to 80% by moles, even more preferably greater than or equal to 90% by moles.

The determination of the degree of deacethylation of chitosan may be carried out according to any method known to the skilled person for this purpose, for example by infrared spectroscopy.

Measurements can be performed in the transmission mode, with chitosan contained in potassium bromide (KBr) tablets. KBr is mixed to chitosan in mass ratio 100:1 (200 mg KBr and 2 mg of chitosan). KBr is placed in an oven at 300 °C for 24 h before mixing. Substances are mixed in agate mortar and pressed to tablet form: three tablets are prepared. The spectra of chitosan samples are obtained within a frequency range of λ = 400-4000 cm⁻¹, each spectrum is an average of 64 scans with a resolution of 2 cm⁻¹. Deacethylation degree is defined as the mole fraction of acethylated units in the mixture.

Chitosans according to the invention are available on the market, for example the product polyglucosamine L112 (produced by Certmedica).

In a further aspect, the present invention relates also to a medical product comprising a chitosan having number average molecular weight in the range from 80 kDa to 350 kDa and at least one organic acid selected from the group consisting of ascorbic acid and tartaric acid, for use in a method of preventing or treating a cardiovascular disease wherein chitosan is a linear polysaccharide composed of randomly distributed β-(1→4)-linked D-glucosamine (deacetylated unit) and N-acetyl-D-glucosamine (acetylated unit.

The Applicant has noted that in a meta-analysis of clinical studies conducted with a particular chitosan in presence of organic acids (ascorbic and tartaric acids), a consistent body weight reduction was documented together with cholesterol reduction (Cornelli U, Belcaro G. Meta-analysis of studies on body weight and cholesterol reduction using the chitosan derivative poluglucosamine L112. Gen Med Open 2018; 2(5):1-4).

The Applicant has found out that by combining the chitosan according to the present invention with at least one organic acid selected from the group consisting of ascorbic acid and tartaric acid a medical product not only capable of reducing cholesterol and body weight, but also capable of reducing the arterial intima media thickness and of controlling the plaque density in blood vessels is obtained.

The Applicant therefore notes that such a medical product provides a further and improved product suitable for preventing or treating a cardiovascular disease, that contributes to reduce the arterial intima media thickness, the plaque density in blood vessels.

The present invention relates therefore to the use of a medical product comprising a chitosan having number average molecular weight in the range from 80 kDa to 350 kDa and at least one organic acid selected from the group consisting of ascorbic acid and tartaric acid, for reducing the arterial intima media thickness and/or for controlling the plaque density in blood vessels, thus providing a product and method for preventing or treating a cardiovascular disease. That is, the present disclosure relates to a method for reducing the arterial intima media thickness and/or to controlling the plaque density in blood vessels comprising the use of a medical product comprising a chitosan having number average molecular weight in the range from 80 kDa to 350 kDa and at least one organic acid selected from the group consisting of ascorbic acid and tartaric acid, wherein chitosan is a linear polysaccharide composed of randomly distributed β-(1→4)-linked D-glucosamine (deacetylated unit) and N-acetyl-D-glucosamine (acetylated unit.

Preferably, said arterial intima media thickness is carotid and/or aorta intima media thickness.

Preferably, said at least one organic acid is present in the medical product in an amount in the range from 5% to 15 % by weight with respect to the chitosan, more preferably in an amount in the range from 7.5 % to 12.5 % by weight with respect ot chitosan.

Preferably, said medical product is administered orally in a solid form comprising a unitary amount of chitosan in the range from 250 to 1000 mg, more preferably in the range from 300 to 750 mg.

Preferably, said medical product is administered so that chitosan is supplied as a daily dose in the range from 1 to 6 g/day, more preferably in the range from 1 to 3 g/day

Preferably, said medical product is administered for a period of at least 3 months. In addition to the components described above, the medical product according to the present invention can advantageously comprise one or more further components preferably selected from the group consisting of: a binder, a stabilizer, a thickener, a solubilizing agent, a lubricant, a disintegrant, an anticacking agent. Preferably, said one or more further components are selected from the group consisting of: povidone, magnesium stearate, sodium croscarmellose, microcrystalline cellulose, silica.

Preferably, the medical product according to the invention may be in the form of a medicament, a medical device, or a food supplement.

Further features and advantages of the invention will appear more clearly from the following description of some preferred embodiments thereof, made hereinafter by way of a non-limiting example with reference to the following exemplary examples.

### EXPERIMENTAL PART

### Material and Methods

The following is the method for determining the number average molecular weight ("Mₙ"): the number average molecular weight was determined by gel-permeation chromatogragy using conventional calibration with pullulan standards, and with the following analysis conditions:
Eluent: 0.1 M NaCl / 0.1% by volume TFA aq.
Columns: PSS NovemaMax 10 µm, guard column, ID 8.0 mm x 50 mm;
   PSS NovemaMax 10 µm, 100 Å, ID 8.0 mm x 300 mm;
   PSS NovemaMax 10 µm, 3000 Å, ID 8.0 mm x 300 mm;
   PSS NovemaMax 10 µm, 3000 Å, ID 8.0 mm x 300 mm
Temperature: 25 °C
Pump: PSS SECcurity 1260 HPLC pump
Flow rate: 0.5 mL / min
Injection System: PSS SECcurity 1260 Autosampler
Injection volume: 100 µL
Sample concentration: approx. 2.0 g / L
Detector: PSS SECcurity 1260 Differential Refractometer RID
Evaluation: PSS WinGPC UniChrom, Version 8.3.1
Sample preparation: the samples were weighed in each case on an analytical balance and mixed with the eluent. The sample concentration was about 2 mg / ml in each case. All sample solutions were swirled at 50 °C overnight. For some samples thereafter, small amounts of insoluble residues were present. Prior to measurement, all sample solutions were filtered through a nominal 1.0 µm PTFE membrane filter. The GPC results refer only to the soluble components.
Calibration and results: various pullulan standards having known molecular weights were measured under the same analytical conditions as the samples, and a conventional calibration was made from the results obtained. All evaluations are based on this calibration. The calculation of the number average molecular weight was computer-aided based on the conventional pullulan calibration curve.

### Example 1

A sample of chitosan with degree of deacethylation of 80 % by moles, marketed as polyglucosamine L112, was provided by Certmedica and characterized in terms of number average molecular weight, according to the method reported in the above paragraph "Material and Method". Figure 1 shows the pullulan calibration curve used for determining the number average molecular weight of the chitosan sample.

The sample of chitosan showed a number average molecular weight of 125 kDa.

The same sample of chitosan was then used to prepare two medical products (respectively "Tablet 1" and Tablet 2") in form of a solid tablet, with composition as reported in Table 1, according to a standard tableting procedure.

**TABLE 1**

| | Tablet 1 | Tablet 2 |
|---|---|---|
| | (mg per tablet) | (mg per tablet) |
| Chitosan | 364.00 | 546.00 |
| Ascorbic acid | 24.00 | 36.00 |
| Tartaric acid | 12.00 | 18.00 |
| Povidone (marketed as Kollidon 30) | 6.00 | 9.00 |
| Magnesium stearate (marketed as Ligamed MF-2-V) | 0.50 | 0.75 |
| Sodium croscarmellose (marketed as Vivasol GF) | 8.50 | 12.75 |
| Microcrystalline cellulose (marketed as Avicel PH 200) | 83.00 | 124.50 |
| Silica (marketed as Aerosil 200) | 2.00 | 3.00 |

### Example 2

In order to test the effects of the chitosan according to the invention on the intima media thickness, on the plaque density and thus for preventing or treating a cardiovascular disease, a trial was carried out.

### Subject involved in the trial, admission and exclusion criteria, group formation

The subjects involved were part of a registry study conducted as part of the San Valentino epidemiological study (PE- Italy) aimed to prevent the cardiovascular risk according to an established Standard Management (SM).

The SM consisted of suggestions about the diet (reduction of NaCl, sugars, fats and carbohydrates) and increase of daily activity (1 h brisk walking/day).

Initially a complete analysis of the food intake was necessary, without any caloric restriction, to identify those foods that may favor the atherosclerosis development.

The concomitant intima media thickness (IMT) control was fundamental to have an objective evaluation of the cardiovascular risk. Te screening process took about three months to determine a relationship between food intake, cholesterol levels and IMT. During this period the subjects entered an observational phase, called registry, which then continued with the adjustment of food intake, physical activity and supplementations with products that may modify cholesterol levels and body weight.

Subjects who completed this first screening period were participating to the study, and were familiar with the Food Intake Assessment evaluation (FIA).

Seventy mildly overweight and hypercholesterolemic subjects (62 males and 8 females) were selected, in the age of 45-60, following the SM.

The admission criteria for the registry were: age between 40 to 55 years, hypercholesterolemia (fasting CH > 240 mg/dL), triglycerides > 170 mg/dL, BMI > 26 and < 30 kg/m2.

The exclusion criteria were chronic diseases such as diabetes, hypertension, cancer, allergy, neurological and immunological disorders. The incapacity to fill up a reliable FIA was also among the exclusion criteria. The reliability of the FIA was measured according to Mifflin-St Jeor algorithm (MSJ). Those subjects resulting with a caloric intake lower than 90 % of the theoretical MSJ values (according to age, height, and weight) were not admitted to the study.

After a preliminary discussion about the possible benefit of the body weight reduction and hypolipidemic management, subjects agreed to enter the study and were divided into 2 groups, each of 35 subjects:
Group 1 (control): 31 males and 4 females (age at the baseline 45.8 ± 2.23 years); and
Group 2: 31 males and 4 females (age at the baseline 46.7 ± 3.22 years).

### Variables investigated

The main variables investigated during the test were body weight, total cholesterol decrease and modification of the ultrasound scanning of carotid and aorta (IMT, intima media thickness).

Total cholesterol, triglycerides, and all the anthropometric measures such as body mass index (BMI), abdominal circumference at the umbilicus (AC), ultrasound scanning for IMT were taken at baseline and after 3 months.

Food Intake Assessment was determined according to a weekly questionnaire consisting of the most common 250 foods of the area, measured as weekly portions. Subjects were instructed how to fill up the questionnaire by a nutritionist. Two sets of evaluation were taken: the baseline evaluation and the one after three months. The quantity of water, proteins, lipids, carbohydrates, fiber, alcohol and the number of weekly portions were measured.

IMT was determined with ultrasound scanning methods that have been already described in detail (Veller MG, Fisher CM, Nicolaides AN, Renton S, Geroulakos G, Stafford NJ. Measurement of the ultrasound intima-media complex thickness in normal subjects. J Vasc Surg 1993;17:719-725; Belcaro G, Barsotti A, Nicolaides AN. "Ultrasonic biopsy" a non invasive screening technique to evaluate the cardiovascular risk and to follow up the progression and the regression of atherosclerosis. Vasa 1991;20:40-50). Both carotid and aorta were scanned using a PREIUS Elastosonographer Hitachi with a linear-array transducer (10-14vMhZ). The carotid and aorta arteries were imaged in transverse and longitudinal planes. Carotid artery examination included evaluation of the common carotid artery within 1.5 cm of the origin of carotid bulb, the carotid bulb itself, and the internal and external carotid arteries. The maximum IMT was measured on the ultrasound image. Plaque were defined as a focal projections of at least 1.5 mm of the arterial wall into the lumen as defined according to previous work and confirmed for reproducibility.

### Treatments

To the subjects of Group 1 it was requested to continue with some limitation of the diets. The suggestion was to reduce two portions/week of foods containing high amount of carbohydrates and/or fats, and to report the modification on the FIA questionnaire. The increase of physical activity was one of the most important element for SM, and the request was 1h of brisk walking/day. This was the typical SM procedure for subjects participating to the registry.

To the subjects of Group 2 it was requested to follow the same SM of Group 1, but with the administration, in addition, of chitosan according to the invention (supplied with "Tablet 1" according to Example 1). The subjects of Group 2 were given 2 boxes of tablets (60 tabs each box) and they received the same amount every month and were asked to take 4 tablets/day, being supplied therefore with 1.456 grams of chitosan per day.

### Blood sampling

Blood sampling was done at baseline (no more than 3 days before the starting of the study) and after 3 months. Blood was drawn from patients after overnight fasting in the quantity of 10 ml from the brachial vein part in EDTA tubes and 0.5 mL in heparinized microvettes. Total cholesterol and triglycerides were measured using the common laboratory methods and dedicated reagents.

### Statistical analysis

For all the data the average and dispersion variable were calculated. T test for interdependent data was calculated to compare baseline values Vs three month. The Anova (with Bonferroni correction) was used to compare the differences between baseline and three months values of the two groups. The correlation coefficients were also determined among IMT and anthropometric/laboratory variables using the ellipse analysis.

### Compliance

The compliance of the subject was measured for the treatment with chitosan, counting every month the remaining tablets in the box, and the cut-off was % of the theoretical quantity to be taken.

### Results

In the case of Group 1, 1 subject was not present at the final control, whereas in the Group 2, 4 subjects abandoned the study for logistic reasons.

The Anthropometric variables, the Food intake assessment profile and the Ultrasound scan and Lab analysis of the subjects of Group 1 and Group 2 at the baseline and after 3 months are reported in Tables 2-4.

The compliance of the cases treated with chitosan was very good and no side effects were reported. None of the subjects was smoking. The two groups were similar for all the variables (t test p<0.05).

**TABLE 2. Anthropometric variables mean values ± SD**

| Variable | Measure | Group 1 | | Group 2 | |
|---|---|---|---|---|---|
| | | baseline | after 3 months | baseline | after 3 months |
| BW | Kg | 87.5 ± 2.11 | 86.5 ± 3.5 ^{a} | 88.4 ± 1.83 | 82.1 ± 3.28^{a b} |
| AC | Cm | 95.6 ± 7.12 | 94.1 ± 4.12 ^{a} | 96.7 ± 3.27 | 91.3 ± 3.14^{a b} |
| BMI | Kg/m2 | 28.1 ± 1.18 | 27.8 ± 4.92 ^{a} | 28.2 ± 0.99 | 26.2 ± 1.26 ^{a b} |

| | | | | | |
|---|---|---|---|---|---|
| a = baseline Vs 3 month - t test for interdependent data p <0.05: b= ANOVA with Bonferroni correction Group 1 Vs Group 2 p < 0.05 | | | | | |

**TABLE 3. Food intake assessment profile:mean values ± SD**

| Variable | Measure | Group 1 | | Group 2 | |
|---|---|---|---|---|---|
| | | baseline | after 3 months | baseline | after 3 months |
| Calories | Kcal | 13879 ± 691.8 | 13214 ± 957.6 | 14111 ± 782.7 | 12225 ± 892.3 ^{a b} |
| N portions | N | 83 ± 7.5 | 81 ± 7.6 ^{a} | 82 ± 6.9 | 74 ± 7.5 ^{a b} |
| Water | L | 14.1 ± 9.10 | 14.0 ± 842.4 | 14.2 ± 9.72 | 13.8 ± 9.0 ^{a b} |
| Protein | g/week | 580 ± 49.7 | 565 ± 53.8 ^{a} | 598 ± 48.9 | 534 ± 44.2 ^{a} |
| Lipids | g/week | 636 ± 47.2 | 609 ± 55.2 ^{a} | 655 ± 58.3 | 597 ± 59.2 ^{a b} |
| Carbohydrates | g/week | 1473 ± 139.2 | 1353 ±213.6 ^{a} | 1480 ± 126.9 | 1205 ± 155.4 ^{a b} |
| Fiber | g/week | 94 ± 10 2 | 89 ± 12.9 ^{a} | 93 ± 9.7 | 99 ± 10.7^{a b c} |
| Alcohol | g/week | 69 ± 66.4 | 66 ± 67.6 | 64 ± 68.2 | 61 ± 66.0 ^{a b} |

| | | | | | |
|---|---|---|---|---|---|
| a = baseline Vs 3 month - t test for interdependent data p <0.05: b= ANOVA with Bonferroni correction Group 1 Vs Group 2 p < 0.05 c = addition of Tablet 2 in the calculations | | | | | |

**TABLE 4. Ultrasound scan and Lab analysis:mean values ± SD**

| Variable | Measure | Group 1 | | Group 2 | |
|---|---|---|---|---|---|
| | | baseline | after 3 months | baseline | after 3 months |
| IMT carotid | mm | 1.224 ± 0.1781 | 1.228 ± 0.2163 | 1.1223 ± 0.2072 | 1.013 ± 0.2056 ^{a b} |
| IMT Aorta | mm | 1.332 ± 0.1211 | 1.335 ± 0.1999 | 1.324 ± 0.1847 | 1.278 ± 0.1531 ^{a b} |
| Intensity | % | 100 | 97.3 ± 1.27 ^{a} | 100 | 93.4 ± 1.27 ^{a b} |
| Plaque density | % | 39.9 ± 6.62 | 37.3 ± 6.12 | 38.4 ± 6.80 | 41.2 ± 3.61 ^{a b} |
| CH | mg/dL | 231.3 ± 10.83 | 208.2 ± 17.98 ^{a} | 237.0 ± 12.04 | 191.7 ± 13.10 ^{a b} |
| Triglycerides | mg/dL | 191.5 ± 11.96 | 184.3 ± 17.8^{a} | 187.9 ± 14.43 | 147.0 ± 11.28 ^{a b} |

| | | | | | |
|---|---|---|---|---|---|
| a = baseline Vs 3 month - t test for interdependent data p <0.05: b= ANOVA with Bonferroni correction Group 1 Vs Group 2 p < 0.05 | | | | | |

### Conclusions

The anthropometric variables were modified in Group 1 in terms of BW, BMI, and AC showing a reduction of 1.1%, 0.9 %, and 1.7 % respectively. Despite the reduction was not large it was statistically significant (t test for interdependent data).

More consistent reductions of the same variables were shown in Group 2, reaching the 7.1 % for BW, 7.1 % for BMI, and 5.5 % for AC.

Comparing the results of the two groups the differences in favor of Group 2 were statistically significant (ANOVA p <0.05), confirming the effectiveness of the medical product comprising chitosan.

The modification of FIA in Group 1 was indicating that water intake and alcohol were not modified, whereas carbohydrates, fiber and proteins were reduced respectively by 8.1 %, 5.6 % and 3.9 %.

The number of weekly portions was 3 % less and the total caloric intake was reduced by 4.8 %.

The use of chitosan was causing more consistent modifications since, a part of fiber increase of 6.2 % - due to the use of chitosan- all the other variables were significantly reduced (p<0.001 t test for interdependent data).

The highest reduction was shown by the carbohydrates intake (19.0 %), followed by proteins (10.7 %), and lipids (8.9 %). Water intake and alcohol were minimally reduced (respectively of 3.9 and 2.9 %). The total caloric intake was 13.4 % less than baseline, and the number of portions was diminished by 9.8 %.

The differences between treatments were statistically significant (ANOVA p< 0.001), again confirming the effectiveness of the medical product comprising chitosan.

The laboratory analysis showed that Group 1 was significantly effective in reducing total CH and triglycerides respectively of 10.0 % and 3.8 % (p< 0,05 t test interdependent data), but also in this case in Group 2 the reduction was much more consistent (respectively 19.8 % and 13.0 %).

The ultrasound scan was not modified in Group 1 in any of the variables, which remained almost similar to the baseline values. Very different picture was shown in Group 2, where a significant reduction of the intima media thickness in both carotid and aorta was noted and in which a stabilization of the plaque was also noted.

It was therefore concluded that the use of chitosan (contained in tablet 2) produced a more consistent reduction of body weight, total cholestero and caloric intake, allowed a reduction of the intima media thickness in both carotid and aorta and a stabilization of plaques, thus providing an improved product suitable for preventing or treating a cardiovascular disease.

## Claims

1. A chitosan having number average molecular weight in the range from 80 kDa to 350 kDa, for use in a method of preventing or treating a cardiovascular disease, wherein chitosan is a linear polysaccharide composed of randomly distributed β-(1→4)-linked D-glucosamine (deacetylated unit) and N-acetyl-D-glucosamine (acetylated unit).

2. A chitosan having number average molecular weight in the range from 80 kDa to 350 kDa, for use in therapeutically reducing the arterial intima media thickness, wherein chitosan is a linear polysaccharide composed of randomly distributed β-(1→4)-linked D-glucosamine (deacetylated unit) and N-acetyl-D-glucosamine (acetylated unit).

3. A chitosan having number average molecular weight in the range from 80 kDa to 350 kDa, for use in therapeutically reducing the plaque density in blood vessels, wherein chitosan is a linear polysaccharide composed of randomly distributed β-(1→4)-linked D-glucosamine (deacetylated unit) and N-acetyl-D-glucosamine (acetylated unit).

4. The chitosan for use according to anyone of claims from 1 to 3, having a degree of deacethylation greater than or equal to 60% by moles.

5. The chitosan for use according to anyone of claims from 1 to 4, having a number average molecular weight in the range from 80 to 265 kDa.

6. The chitosan for use according to claim 5, having a number average molecular weight in the range from 80 kDa to 250 kDa.

7. A medical product comprising a chitosan having number average molecular weight in the range from 80 kDa to 350 kDa and at least one organic acid selected from the group consisting of ascorbic acid and tartaric acid, for use in a method of preventing or treating a cardiovascular disease, wherein chitosan is a linear polysaccharide composed of randomly distributed β-(1→4)-linked D-glucosamine (deacetylated unit) and N-acetyl-D-glucosamine (acetylated unit).

8. The medical product for use according to claim 7, wherein the chitosan has a number average molecular weight in the range from 80 to 265 kDa.

9. The medical product for use according to claim 8, wherein the chitosan has a number average molecular weight in the range from 80 kDa to 250 kDa.

10. The medical product for use according to anyone of claims from 7 to 9, wherein said at least one organic acid is present in an amount in the range from 5% to 15 % by weight with respect to the chitosan.

11. The medical product for use according to anyone of claims from 7 to 10, wherein said medical product is administered orally in a solid form comprising a unitary amount of chitosan in the range from 250 to 1000 mg.

12. The medical product for use according to any one of claims from 7 to 11, wherein said medical product is administered so that chitosan is supplied as a daily dose of 1 to 6 g/day.

13. The medical product for use according to any one of claims from 7 to 12, wherein said medical product is administered for a period of at least 3 months.

14. The medical product for use according to any one of claims from 7 to 13, wherein the medical product is selected from the group consisting of a medicament, a medical device and a food supplement.

15. The chitosan for use according to any one of claims from 1 to 6 or the medical product for use according to any one of claims from 7 to 14, wherein said chitosan is used as a product for reducing arterial intima media thickness and/or for reducing the plaque density in blood vessels.

16. The chitosan or the medical product for use according to claim 15, wherein said arterial intima media thickness is carotid and/or aorta intima media thickness.

## Patentansprüche

1. Chitosan, das ein zahlenmittleres Molekulargewicht im Bereich von 80 kDa bis 350 kDa aufweist, zur Verwendung in einem Verfahren zur Vorbeugung oder Behandlung einer kardiovaskulären Erkrankung, wobei das Chitosan ein lineares Polysaccharid ist, das aus zufällig verteiltem β-(1→4)-verknüpftem D-Glucosamin (deacetylierte Einheit) und N-Acetyl-D-Glucosamin (acetylierte Einheit) besteht.

2. Chitosan, das ein zahlenmittleres Molekulargewicht im Bereich von 80 kDa bis 350 kDa aufweist, zur Verwendung bei der therapeutischen Verringerung der arteriellen Intima-Media-Dicke, wobei das Chitosan ein lineares Polysaccharid ist, das aus zufällig verteiltem β-(1→4)-verknüpftem D-Glucosamin (deacetylierte Einheit) und N-Acetyl-D-Glucosamin (acetylierte Einheit) besteht.

3. Chitosan, das ein zahlenmittleres Molekulargewicht im Bereich von 80 kDa bis 350 kDa aufweist, zur Verwendung bei der therapeutischen Verringerung der Plaque-Dichte in Blutgefäßen, wobei das Chitosan ein lineares Polysaccharid ist, das aus zufällig verteiltem β-(1→4)-verknüpftem D-Glucosamin (deacetylierte Einheit) und N-Acetyl-D-Glucosamin (acetylierte Einheit) besteht.

4. Chitosan zur Verwendung nach einem der Ansprüche 1 bis 3, das einen Deacetylierungsgrad von größer oder gleich 60 mol-% aufweist.

5. Chitosan zur Verwendung nach einem der Ansprüche 1 bis 4, das ein zahlenmittleres Molekulargewicht im Bereich von 80 bis 265 kDa aufweist.

6. Chitosan zur Verwendung nach Anspruch 5, das ein zahlenmittleres Molekulargewicht im Bereich von 80 kDa bis 250 kDa aufweist.

7. Medizinisches Produkt, das ein Chitosan, das ein zahlenmittleres Molekulargewicht im Bereich von 80 kDa bis 350 kDa aufweist, und mindestens eine organische Säure, ausgewählt aus der Gruppe, bestehend aus Ascorbinsäure und Weinsäure, umfasst, zur Verwendung in einem Verfahren zur Vorbeugung oder Behandlung einer kardiovaskulären Erkrankung, wobei das Chitosan ein lineares Polysaccharid ist, das aus zufällig verteiltem β-(1→4)-verknüpftem D-Glucosamin (deacetylierte Einheit) und N-Acetyl-D-Glucosamin (acetylierte Einheit) besteht.

8. Medizinisches Produkt zur Verwendung nach Anspruch 7, wobei das Chitosan ein zahlenmittleres Molekulargewicht im Bereich von 80 bis 265 kDa aufweist.

9. Medizinisches Produkt zur Verwendung nach Anspruch 8, wobei das Chitosan ein zahlenmittleres Molekulargewicht im Bereich von 80 kDa bis 250 kDa aufweist.

10. Medizinisches Produkt zur Verwendung nach einem der Ansprüche 7 bis 9, wobei die mindestens eine organische Säure in einer Menge im Bereich von 5 Gew.-% bis 15 Gew.-%, bezogen auf das Chitosan, vorhanden ist.

11. Medizinisches Produkt zur Verwendung nach einem der Ansprüche 7 bis 10, wobei das medizinische Produkt oral in fester Form verabreicht wird, die eine einheitliche Menge an Chitosan im Bereich von 250 bis 1000 mg umfasst.

12. Medizinisches Produkt zur Verwendung nach einem der Ansprüche 7 bis 11, wobei das medizinische Produkt so verabreicht wird, dass Chitosan in einer Tagesdosis von 1 bis 6 g/Tag zugeführt wird.

13. Medizinisches Produkt zur Verwendung nach einem der Ansprüche 7 bis 12, wobei das medizinische Produkt über einen Zeitraum von mindestens 3 Monaten verabreicht wird.

14. Medizinisches Produkt zur Verwendung nach einem der Ansprüche 7 bis 13, wobei das medizinische Produkt aus der Gruppe ausgewählt ist, die aus einem Arzneimittel, einer medizintechnischen Ausrüstung und einem Nahrungsergänzungsmittel besteht.

15. Chitosan zur Verwendung nach einem der Ansprüche 1 bis 6 oder medizinisches Produkt zur Verwendung nach einem der Ansprüche 7 bis 14, wobei das Chitosan als Produkt zur Verringerung der arteriellen Intima-Media-Dicke und/oder zur Verringerung der Plaque-Dichte in Blutgefäßen verwendet wird.

16. Chitosan oder medizinisches Produkt zur Verwendung nach Anspruch 15, wobei die arterielle Intima-Media-Dicke die Karotis- und/oder Aorta-Intima-Media-Dicke ist.

## Revendications

1. Chitosane ayant un poids moléculaire moyen en nombre compris entre 80 kDa et 350 kDa, destiné à être utilisé dans un procédé de prévention ou de traitement d'une maladie cardiovasculaire, dans lequel le chitosane est un polysaccharide linéaire composé de D-glucosamine (unité désacétylée) et de N-acétyl-D-glucosamine (unité acétylée) liées en β-(1→4) distribuées de manière aléatoire.

2. Chitosane ayant un poids moléculaire moyen en nombre compris entre 80 kDa et 350 kDa, destiné à être utilisé dans la réduction thérapeutique de l'épaisseur intima-média des artères, dans lequel le chitosane est un polysaccharide linéaire composé de D-glucosamine (unité désacétylée) et de N-acétyl-D-glucosamine (unité acétylée) liées en β-(1→4) distribuées de manière aléatoire.

3. Chitosane ayant un poids moléculaire moyen en nombre compris entre 80 kDa et 350 kDa, destiné à être utilisé dans la réduction thérapeutique de la densité de la plaque dans les vaisseaux sanguins, dans lequel le chitosane est un polysaccharide linéaire composé de D-glucosamine (unité désacétylée) et de N-acétyl-D-glucosamine (unité acétylée) liées en β-(1→4) distribuées de manière aléatoire.

4. Chitosane destiné à être utilisé selon l'une quelconque des revendications 1 à 3, ayant un degré de désacétylation supérieur ou égal à 60 % en moles.

5. Chitosane destiné à être utilisé selon l'une quelconque des revendications 1 à 4, ayant un poids moléculaire moyen en nombre compris entre 80 et 265 kDa.

6. Chitosane destiné à être utilisé selon la revendication 5, ayant un poids moléculaire moyen en nombre compris entre 80 kDa et 250 kDa.

7. Produit médical comprenant un chitosane ayant un poids moléculaire moyen en nombre compris entre 80 kDa et 350 kDa et au moins un acide organique choisi dans le groupe constitué par l'acide ascorbique et l'acide tartrique, destiné à être utilisé dans un procédé de prévention ou de traitement d'une maladie cardiovasculaire, dans lequel le chitosane est un polysaccharide linéaire composé de D-glucosamine (unité désacétylée) et de N-acétyl-D-glucosamine (unité acétylée) liées en β-(1→4) distribuées de manière aléatoire.

8. Produit médical destiné à être utilisé selon la revendication 7, dans lequel le chitosane a un poids moléculaire moyen en nombre compris entre 80 et 265 kDa.

9. Produit médical destiné à être utilisé selon la revendication 8, dans lequel le chitosane a un poids moléculaire moyen en nombre compris entre 80 kDa et 250 kDa.

10. Produit médical destiné à être utilisé selon l'une quelconque des revendications 7 à 9, dans lequel ledit au moins un acide organique est présent en une quantité comprise entre 5 % et 15 % en poids par rapport au chitosane.

11. Produit médical destiné à être utilisé selon l'une quelconque des revendications 7 à 10, dans lequel ledit produit médical est administré par voie orale sous une forme solide comprenant une quantité unitaire de chitosane comprise entre 250 et 1000 mg.

12. Produit médical destiné à être utilisé selon l'une quelconque des revendications 7 à 11, dans lequel ledit produit médical est administré de sorte que le chitosane soit fourni en une dose quotidienne de 1 à 6 g/jour.

13. Produit médical destiné à être utilisé selon l'une quelconque des revendications 7 à 12, dans lequel ledit produit médical est administré pendant une période d'au moins 3 mois.

14. Produit médical destiné à être utilisé selon l'une quelconque des revendications 7 à 13, dans lequel le produit médical est choisi dans le groupe constitué d'un médicament, d'un dispositif médical et d'un complément alimentaire.

15. Chitosane destiné à être utilisé selon l'une quelconque des revendications 1 à 6 ou produit médical destiné à être utilisé selon l'une quelconque des revendications 7 à 14, dans lequel ledit chitosane est utilisé comme produit pour réduire l'épaisseur intima-média des artères et/ou pour réduire la densité de la plaque dans les vaisseaux sanguins.

16. Chitosane ou produit médical destiné à être utilisé selon la revendication 15, dans lequel ladite épaisseur intima-média des artères est une épaisseur intima-média carotidienne et/ou aortique.
